# EUROPEAN PATENT SPECIFICATION

(11) **EP 0 676 221 B1**
(45) Date of publication and mention of the grant of the patent: **23.12.1998**
(21) Application number: 95302245.6
(22) Date of filing: 04.04.1995
(51) Int. Cl.: A62D 3/00, C07C 45/53

(54) **Dried catalytic systems for decomposition of organic hydroperoxides**
Getrocknetes Katalysatorsysteme zur Zersetzung von organischen Hydroperoxiden
Systèmes catalytiques déshydratés pour la décomposition d'hydroperoxydes organiques

(30) Priority: 04.04.1994 US 223090; 03.03.1995 US 398024
(43) Date of publication of application: 11.10.1995
(73) Proprietor: SUN COMPANY, INC. (R&M), Philadelphia, PA 19103-1699 (US)
(72) Inventor: Bhinde, Manoj V., Boothwyn, PA 19061 (US); Lyons, James E., Wallingford, PA 19086 (US); Ellis, Paul E., Jr., Downingtown, PA 19335 (US)
(74) Representative: Lewin, John Harvey

(56) References cited:
- EP-A- 0 308 101
- EP-A- 0 522 841
- US-A- 4 551 553

## Description

### BACKGROUND

Metallophthalocyanine complexes are known catalysts for the decomposition of hydroperoxides. Sanderson et al., U.S. Patents 4,912,266 and 4,912,267, issued March 27, 1990; and 4,922,035 and 4,922,036, issued May 1, 1990, disclose decomposition of t-butyl hydroperoxide (TBHP) dissolved in t-butyl alcohol (TBA, t-butanol) over a metallophthalocyanine catalyst modified by imidazole and other modifiers. Sanderson et al., U.S. Patent 4,992,602, issued February 12, 1991, disclose partial oxidation of isobutane and distillation of the reaction product to obtain a fraction containing 80 to 90% of t-butyl hydroperoxide and 20 to 10% of t-butanol, dissolving that fraction in 3 to 10 parts by weight, based on the weight of the fraction, of benzene, and decomposing the hydroperoxide in the resulting solution with a phthalocyanine decomposition catalyst.

Taylor et al., U.S. Patent 4,551,553, issued November 5, 1985, disclose use of various metal acetylacetonate catalysts, including ruthenium, chromium, cobalt and manganese acetylacetonates, for decomposition of hydroperoxides in undried reaction systems. A catalyst comprising a combination of chromium and ruthenium acetylacetonates was found to have the highest reaction rate in such undried systems.

Lyons and Ellis, U.S. Patent 5,120,886, issued June 9, 1992, disclose and claim decompositions of hydroperoxides by contact with metal ligand catalysts of coordination complexes, including phthalocyanine ligands, in which hydrogen in the phthalocyanine molecule has been substituted with electron-withdrawing elements or groups, for example halogen or nitro or cyano groups. Other ligands than phthalocyanines can be used, for example porphyrins and others as subsequently disclosed.

### SUMMARY OF THE INVENTION

The invention provides a method for decomposing organic hydroperoxides which comprises drying a reaction mixture comprising an organic hydroperoxide, an organic solvent for said hydroperoxide, and water, or drying the organic hydroperoxide or the organic solvent, thereby to obtain a dried reaction mixture in which said water comprises 1 weight percent or less of said dried reaction mixture, and contacting said dried reaction mixture with a metal organic ligand catalyst for said decomposing.

The invention comprises a method for decomposition of organic hydroperoxides to hydroxyl compounds such as alcohols. The use of a dried solution of hydroperoxide in solvent results in an increased decomposition rate of the hydroperoxide, compared with the decomposition rate obtained using a hydroperoxide solution which has not been dried.

Suitable catalysts include cobalt or ruthenium acetylacetonates, metalloporphyrins, metallophthalocyanines and others as subsequently disclosed. Reducing the water content of the reaction mixture to 1 weight percent or less has been found to significantly promote the decomposition rate, compared with the decomposition rate obtained using a hydroperoxide solution which has not been dried to such a degree.

### DESCRIPTION OF THE FIGURES

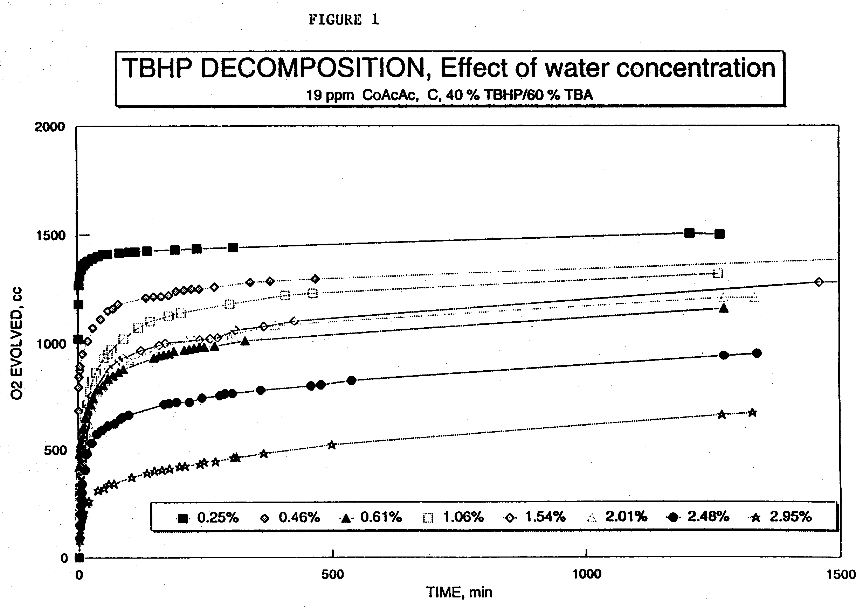

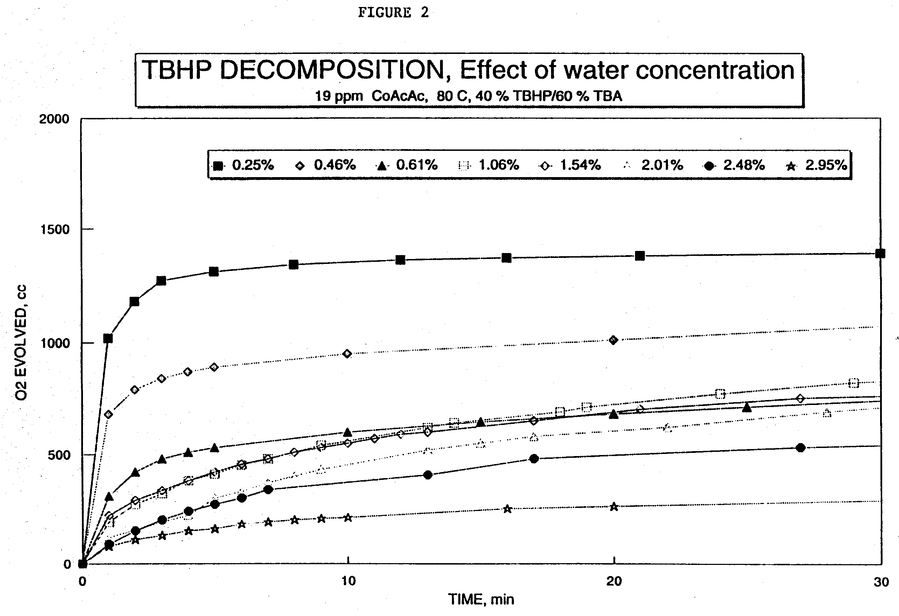

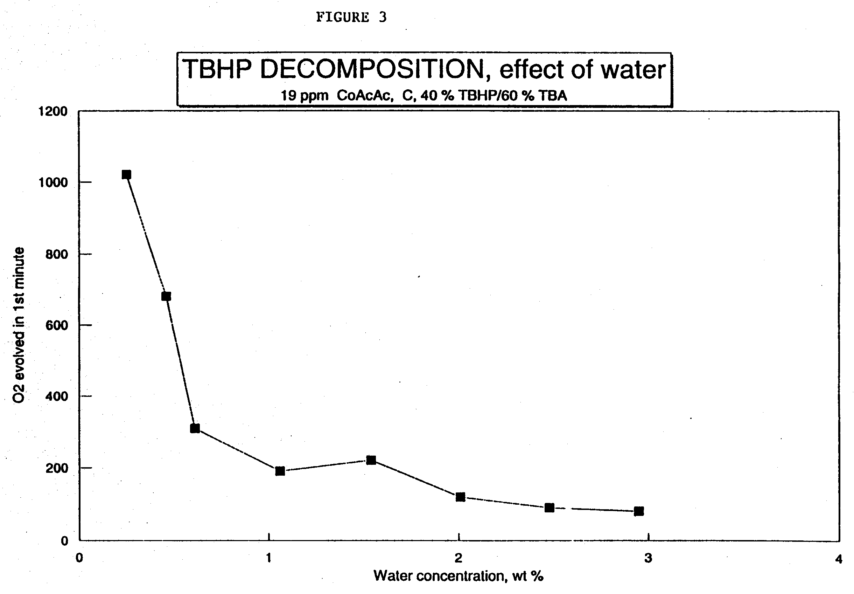

Figures 1 and 2 show the effect of water concentration on the decomposition of t-butyl hydroperoxide in t-butyl alcohol catalyzed by cobalt acetylacetonate (Co(acac)₂), as measured by the amount of O₂ evolved (cc) over time (min). Figure 3 shows the same effect as measured by the O₂ evolved in the first minute of the reaction as a function of the water concentration (in weight percent) in the reaction mixture. Figure 3 is an approximate indication of the relative initial reaction rates as a function of water concentration.

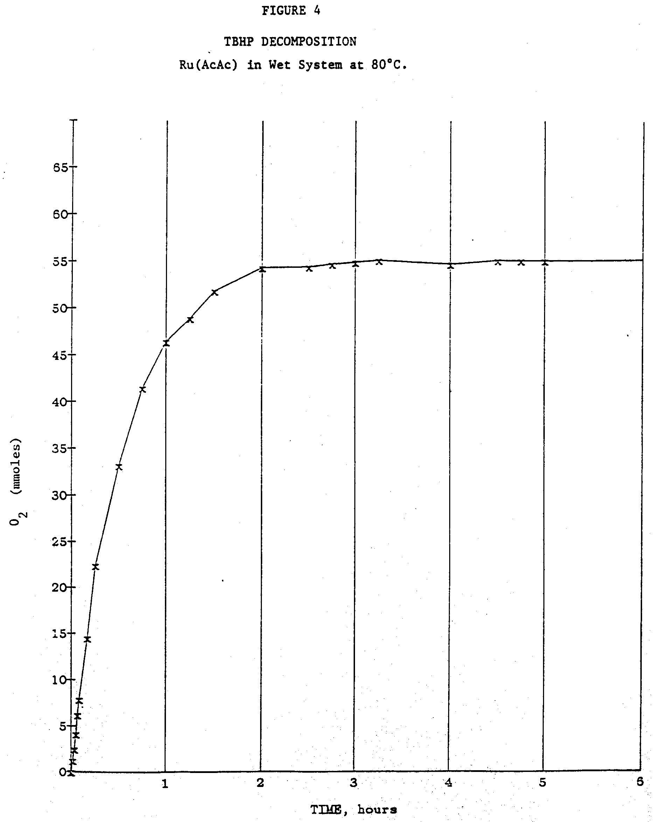

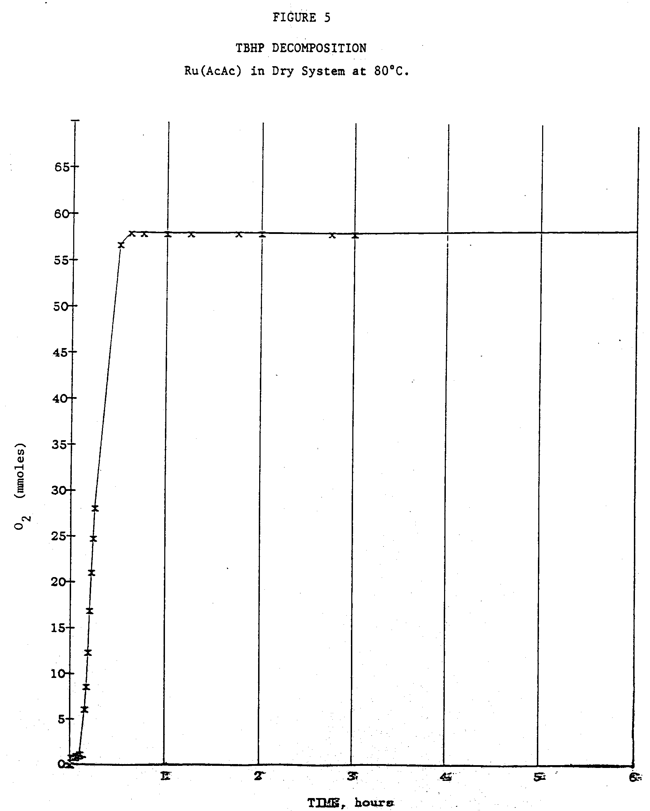

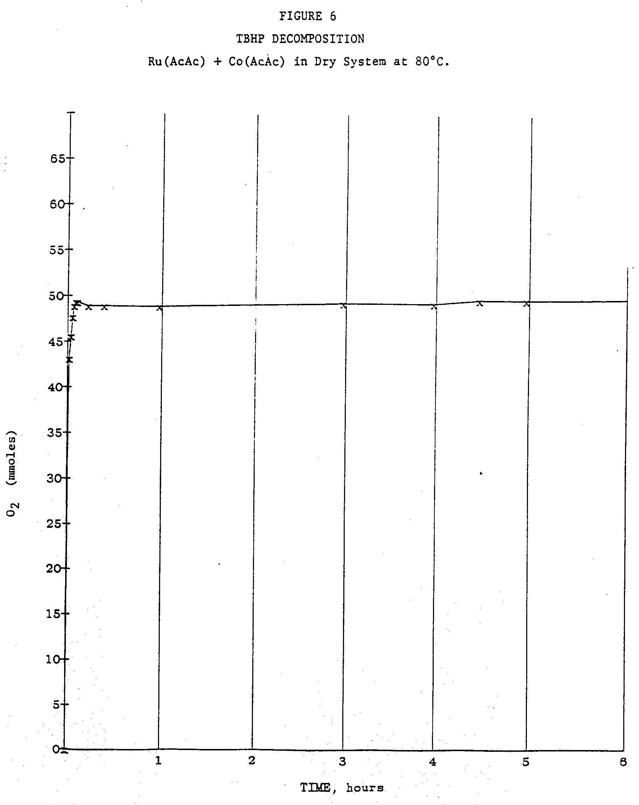

Figures 4 and 5 together show the effect of water concentration on the decomposition of t-butyl hydroperoxide in t-butyl alcohol catalyzed by ruthenium acetylacetonate (Ru(acac)₃) in wet and dry systems, respectively, as measured by the amount of O₂ evolved (mmoles) over time (hours). Figure 6 shows the decomposition of TBHP catalyzed by the combination of Co(acac)₂ and Ru(acac)₃ in a dried system.

### DETAILED DESCRIPTION OF THE INVENTION

The invention comprises a method for decomposition of organic hydroperoxides to hydroxyl compounds such as alcohols which comprises drying a solution of an organic hydroperoxide in at least one organic solvent for the hydroperoxide such that said dried reaction mixture comprises 1 weight percent or less of water, and contacting the dried reaction mixture with a metal organic ligand catalyst. Suitable catalysts include cobalt or ruthenium acetylacetonates, metalloporphyrins, metallophthalocyanines and others as subsequently disclosed. Reducing the water content of the reaction mixture to 1 weight percent or less has been found to significantly promote the decomposition rate, compared with the decomposition rate obtained using a hydroperoxide solution which has not been dried to such a degree. The invention further comprises decomposing reaction mixtures which have been previously dried or which otherwise comprise 1 wt.% or less of water.

"Drying" means reducing the water content of the hydroperoxide and/or the solvent. Thus, the hydroperoxide may be dried, then dissolved in the solvent, or the solvent may be dried and the hydroperoxide then dissolved in it, or a solution of hydroperoxide in the solvent may be dried, or dried hydroperoxide and dried solvent may be combined. Where a co-solvent is used as described below, the co-solvent may be dried together with or separately from the other components of the reaction mixture, in generally similar fashion. A drying step may also be applied to the solution comprising the catalyst, for example catalyst in acetone.

### DRYING OF HYDROPEROXIDE AND/OR SOLVENT

The drying step in the method of the invention may be accomplished by any suitable means for removal of water from organic compounds, such as contact with granular solid drying agents, such as molecular sieves, alumina, silica gel, clays, MgSO₄ and the like, azeotropic distillation, contact with organic drying agents such as ethyl orthoformate, preferably regenerable organic drying agents such as diethyl acetals or ketals, for example dimethylacetal, diethylacetal, di t-butyl acetate, 2,2-dialkoxypropanes such as 2,2-dimethoxypropane and the like. Any substantial drying of the hydroperoxide and/or solvent or solvents, such as which reduces the water content of the reaction mixture to 1 weight percent or less, is within the scope of the invention. Preferably, the water content is 0.5 wt.% or less, more preferably 0.25 wt.%, and still more preferably 0.1 wt.%. Generally, the greater the extent of the drying, up to total or substantially total drying, the greater the beneficial effect on the decomposition rate of the hydroperoxide.

### USE OF CO-SOLVENT

In one embodiment of the invention, the solvent as previously disclosed, hereinafter sometimes referred to as the primary solvent, is used in conjunction with a co-solvent. Such co-solvent is a solvent different from the primary solvent, which may be added to the hydroperoxide and primary solvent to form a solution of hydroperoxide in the primary solvent and co-solvent. Ketones, such as acetone, methylethylketone, methylisobutylketone, C₆-C₁₂ cyclic ketones e.g. cyclohexanone and the like, function effectively as co-solvents, resulting in increased rates of decomposition of hydroperoxides, as compared with the use of the primary solvent without a co-solvent, or as compared with the use of either the primary solvent or the co-solvent alone.

Various types of solvents, for example ketones, are capable of functioning either as primary solvents or as co-solvents. When two solvents are used in conjunction, with one solvent present in larger concentration than the other, the one used in the larger concentration is conveniently considered the primary solvent and the other the co-solvent. If they are present in equal amounts, either may be considered the primary solvent. Typically the proportions of primary solvent and co-solvent will be in the range from 1 to 100 parts by volume of co-solvent per 100 parts of primary solvent, but this depends upon the solubility characteristics of a particular pair of solvents in relation to a particular hydroperoxide, and the optimum proportions may be determined by a person skilled in the art in the light of the present specification. The use of more than two solvents is within the scope of the invention.

### HYDROPEROXIDES

Hydroperoxides for decomposition according to the invention include compounds having the formula ROOH where R is an organic radical, typically a straight or branched chain alkyl group or cycloalkyl group containing 2 to 15 carbon atoms, an aryl group such as a monocyclic or polycyclic group in which the cyclic groups may optionally be substituted with one or more substituents inert to the decomposition reaction, such as alkyl or alkoxy, containing 1 to 7 carbon atoms, nitro, carboxyl or carboxyl ester containing up to 15 carbon atoms and a halogen atom such as chloride, bromide or an alkylaryl group in which the alkyl chain contains 1 to 15 carbon atoms and the aryl group is as above described. Preferably, R is an alkyl or cycloalkyl group containing 4 to 12 carbon atoms or an alkylaryl group in which the aromatic moiety is phenyl and the alkyl group is straight or branched chain alkyl or cycloalkyl containing up to 6 carbon atoms.

Examples of hydroperoxides for decomposition according to the invention are t-butyl and isobutyl hydroperoxide, isoamyl hydroperoxide, t-amylhydroperoxide, cyclohexyl hydroperoxide, alpha- and beta-ethylbenzene hydroperoxide, cumyl hydroperoxide, phenethyl hydroperoxide and cyclohexylphenyl hydroperoxide; phenethyl hydroperoxide and cumyl hydroperoxide are converted to phenethyl alcohol and cumyl alcohol respectively. Preferred are the alkyl hydroperoxides such as t-butyl hydroperoxide, isoamyl hydroperoxide, and the like, and the cycloalkyl hydroperoxides such as cyclohexyl hydroperoxide, methylcyclohexyl hydroperoxide, and the like.

### HYDROPEROXIDE DECOMPOSITION CATALYSTS

Hydroperoxide decomposition catalysts for use according to the invention comprise the transition metal complexes of ligands such as phthalocyanines, porphyrins, porphenes, porphycenes, acetylacetonates, 1,3-bis(arylimino)isoindolines such as "BPI", Schiff bases such as salen, saleph and the like, halogenated mono-, bi-, tri- and tetradentate ligand systems such as propanates, butyrates, benzoates, naphthenates, stearates, bipyridines, terpyridines, phenanthrolines, dithiocarbamates, xanthates, salicylaldimines, cyclam, dioxycyclams, pyrazolyl borates and tetraazamacrocycles such as tetramethyltetraazadibenzocycloheptadecane. Suitable metals for the catalysts useful in the present invention include chromium, manganese, iron, cobalt and ruthenium. In one embodiment of the invention, preferred are the ligands having the hydrogen atoms of the molecule substantially completely replaced with electron-withdrawing atoms or groups such as halogen, nitro, cyano, halocarbyl, nitrocarbyl, cyanocarbyl and the like. The catalysts useful in the process of the present invention may be produced by conventional methods known in the art.

As noted above, the process of the present invention results in a surprising and unexpectedly great increase in the decomposition rate of organic hydroperoxides. An advantage of the present invention is that inexpensive and readily available compositions, which previously were completely or essentially inactive as catalysts, exhibit enhanced activity when utilized in the present invention. Examples of such catalysts are unsubstituted metallophthalocyanines and the metal acetylacetonates, such as cobalt acetylacetonate (Co(acac)₂) and ruthenium acetylacetonate (Ru(acac)₃). For example, Co(acac)₂ has no catalytic activity for decomposition of t-butyl hydroperoxide when the reaction system is contaminated with 5 wt.% water. In contrast, when there is less than 1 wt.% water in the reaction mixture, the same composition is a highly active catalyst. Figures 1 to 3 illustrate this result.

In one embodiment of the invention, the catalyst is a mixture of metallo-phthalocyanine and metal acetylacetonate, for example, iron phthalocyanine (FePc) and Co(acac)₂. In another, the catalyst is a mixture of ruthenium acetylacetonate and cobalt acetylacetonate; the former appears to produce a higher overall conversion rate while the latter exhibits a shorter induction period and greater initial reaction rate. The weight ratio of Ru(acac)₃ to Co(acac)₂ may be from 0.01:1 to 100:1 (Ru:Co), preferably 0.1:1 to 10:1, more preferably 0.1:1 to 1:1. The relative proportion of Co(acac)₂ to Ru(acac)₃ may be modified to balance reaction rate, conversion rate and catalyst cost. Effecting the desired balance is within the ability of the skilled practitioner in the art.

### PREPARATION OF REACTION MIXTURE

The decomposition catalyst may either be dissolved or slurried in the reaction mixture; i.e., in the primary solvent, or if a co-solvent is used, in the co-solvent, or a combination thereof. The effectiveness of the catalyst may be improved by stirring the catalyst in the solvent or co-solvent for at least 24, and up to 168 or more hours. This time can be reduced by heating the slurry, sonication or other devices known to accelerate both solution and dispersion phenomena. The catalyst slurry may be mixed with a solution of the hydroperoxide in the primary solvent, and the resulting mixture or solution subjected to the reaction temperature as subsequently described. The catalyst may be added incrementally to the reaction mixture.

### SOLVENTS AND DECOMPOSITION CONDITIONS

The decomposition of hydroperoxides according to the invention is typically carried out in a solution of the hydroperoxide in the solvent or solvent mixture. The solution preferably contains from about 5 to about 50 wt.% of hydroperoxide, but may also contain less, for instance 1 wt.% of hydroperoxide. Suitable solvents or co-solvents include benzene, chlorobenzene, o-dichlorobenzene, acetonitrile, benzonitrile, hydroxyl or carbonyl compounds such as alcohols or ketones and the like. A useful solvent is the alcohol formed by decomposition of the hydroperoxide, for example TBA formed by decomposition of TBHP. Any suitable temperature and pressure may be used. Preferably the temperature is in the range from 25° to 150°C, preferably to 130°C, and the total pressure from 0 to 3.45 MPa (0 to 500 psig); preferably, the partial pressure of the oxygen in the solution is not more than 345 kPa (50 psig). The time of reaction may be relatively short, in view of the rapid reaction rate with the catalysts employed according to the invention, and will typically be in the range from 0.1 to 5 hours. The drying of the reaction mixture according to the invention may be effected before contacting the hydroperoxide and solvent or solvents with the decomposition catalyst, or it may be effected *in situ* in the decomposition reaction.

### DRYING AND USE OF CO-SOLVENT

The drying of the reaction mixture according to the invention is applicable to reaction mixtures which contain one solvent, and to reaction mixtures which contain a primary solvent and one or more co-solvents. The use of one or more co-solvents according to the invention is applicable to reaction mixtures which are dried, and to reaction mixtures which are not dried. In a preferred embodiment, the reaction mixture is dried and contains one or more co-solvents.

### INTEGRATION OF OXIDATION AND HYDROPEROXIDE DECOMPOSITION

The hydroperoxide which is decomposed according to the invention may have been obtained from any source. Where the hydroperoxide has been prepared by a noncatalytic or catalytic partial oxidation of hydrocarbons, the decomposition of the hydroperoxide may conveniently be integrated with the previous oxidation. The product mixture from the oxidation typically contains unreacted hydrocarbon, hydroperoxide and alcohol products of the partial oxidation, and water, and is typically fractionated to obtain a fraction comprising a water-containing solution of hydroperoxide in the alcohol product of the oxidation. In the integrated operation, the water-containing oxidation product or a water-containing fraction thereof, is passed to a drying operation wherein the water content is reduced. The product of the drying operation is passed to the decomposition of hydroperoxide according to the invention. The use of a drier unit prior to the hydroperoxide decomposition (HPD) unit allows for a substantial increase in HPD reactor throughput due to the increased reaction rate of the process of the invention, possibly as much as an order of magnitude or more. Alternatively, the drying operation may be carried out in the decomposition reactor itself, concurrently with the decomposition, rather than in a separate, prior step.

### COMMERCIAL PRODUCTION OF t-BUTANOL

In the commercial noncatalytic production of t-butanol ("TBA") by partial oxidation of isobutane, a mixture of TBA and TBHP is typically obtained as reaction product. The TBHP is often reacted in a second step with an olefin to produce an alkylene oxide and TBA as products. The invention provides additional options in connection with such operation which may be advantageous in some cases. Thus, for example, the partial oxidation may be conducted either catalytically or noncatalytically to maximize TBHP production relative to TBA production, and the method of the invention used to convert the TBHP produced to TBA in a manner to maximize the overall production of TBA independently of any other product.

### DRIED HYDROPEROXIDE AND ORGANIC SOLVENT COMPOSITIONS

Related to the invention are compositions of matter, useful in the methods of the invention, which comprise solutions of an organic hydroperoxide in an organic solvent, prepared by:
(a) drying the hydroperoxide to obtain a dried hydroperoxide and mixing the dried hydroperoxide with the dried or undried solvent,
(b) drying the solvent and mixing the dried solvent with the dried or undried hydroperoxide,
(c) drying a mixture of the hydroperoxide and the solvent, or
(d) drying a product mixture from the partial oxidation of alkane that comprises hydroperoxide together with other by-products.

Preferably, the hydroperoxide-solvent or hydroperoxide-solvent-cosolvent composition of the invention contains less than 50%, and more preferably less than 25%, of the water in the corresponding undried composition, that is, the composition consisting of the same relative portions of the same components in their undried state. These compositions also preferably contain approximately 1 weight percent or less, preferably 0.5 wt.% or less, more preferably 0.25 wt.% or less, and still more preferably 0.1 wt.% or less of water. In contrast, typical commercial feeds may contain 2 to 5 wt.% or more of water. For example, where the undried composition contains 2 wt.% water, and the dried composition contains 0.05 wt.% water, the dried composition contains (0.05/2)x100, or 2.5%, of the water in the undried composition.

### EXAMPLES

The following examples illustrate the invention.

### Examples 1 through 12

Table I shows the effect of ketonic co-solvent on the decomposition of TBHP, solubilized in t-butyl alcohol ("TBA"), and catalyzed by metal phthalocyanine. TBHP was decomposed in Examples 1 to 12 using metal phthalocyanine as catalyst, and the effect of the addition of ketonic co-solvent was determined by using varying conditions in the twelve runs. The procedure used was as described in footnote a to Table I. The twelve lines of data in Table I are Examples 1 to 12.

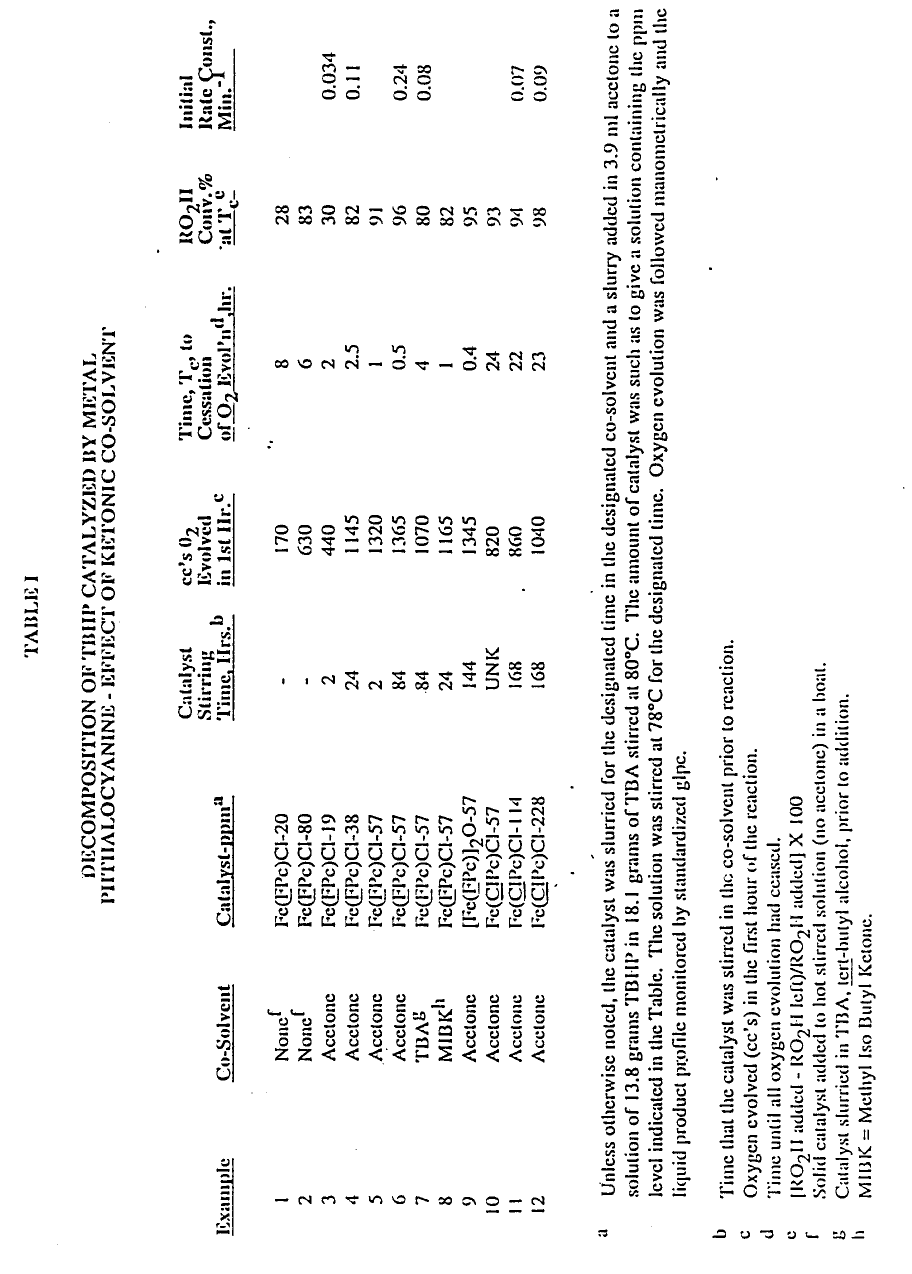

The data under the heading "cc's O₂ Evolved in 1st Hr" in Table I indicate the relative effectiveness of the runs. For example, the 1145 cc's of oxygen evolved in the first hour in Example 4 indicates a more effective decomposition than that obtained in Example 3, where the corresponding amount was 440. In Example 6, using acetone as co-solvent and 57 ppm of Fe^{III} perfluoro (F) phthalocyanine (Pc) chloride (Cl) as catalyst, 1365 cc's of oxygen were evolved in the first hour. In Example 6, the catalyst was stirred in the acetone co-solvent for 84 hours prior to contacting the TBHP with the catalyst solution. All oxygen evolution had ceased after 0.5 hour of the reaction, at which time 96 % of the TBHP ("RO₂H") had been converted, principally to ROH, where R is t-butyl.

Comparison of Examples 4 through 12 with Examples 1 through 3 shows increased oxygen evolution with at least 38 ppm of catalyst and using ketone as co-solvent, compared with Examples 1 and 2 where no co-solvent was used and Example 3 where acetone was used as co-solvent with only 19 ppm of catalyst.

### Examples 13 through 15

Table II shows the effect of added acetone on the decomposition of TBHP using 90, 59 and 59 ppm of Fe[FPc]Cl, the same catalyst as in Examples 1 to 12, at a temperature of 80°C. and pressure of 1.38 MPa (200 psig), compared with 80°C. and atmospheric pressure in Examples 1 to 12.

Table II shows the superiority of the method of the invention, in Examples 14 and 15, using acetone as co-solvent, to Example 13, using no co-solvent. Examples 14 and 15 achieved similar results, in terms of % conversion of TBHP, to the results of Example 13, with a smaller catalyst concentration (59 v 90 ppm) and a shorter reaction time 180/195 minutes v 335 minutes).

**TABLE II**

| **TBHP DECOMPOSITION Effect of added Acetone Fe[FPc]Cl catalyst @ 80 C** | | | | | | | |
|---|---|---|---|---|---|---|---|
| **EXAMPLE #** | **RUN #** | **CAT CONC ppm** | **% CONV of TBHP** | **TIME min** | **PRESSURE psig** | **TBHP Charge mole %** | **Acetone Mole %** **Actual** |
| 13 | 3905a | 90 | 78.4 | 335 | 200 * | 29.24 | 0 |
| 14 | 3911b | 59 | 72.4 | 195 | 200 * | 31.80 | 11.6 |
| 15 | 3925c | 59 | 79.6 | 180 | 200 * | 31.68 | 11.6 |

| | | | | | | | |
|---|---|---|---|---|---|---|---|
| a Catalyst (slurried in TBA) heated to 80 C; TBHP added from reservoir, no acetone. | | | | | | | |
| b Catalyst (slurried in TBA) + 1.2 g acetone heated to 80 C; TBHP added from reservoir; 8.7 wt % total acetone. | | | | | | | |
| c Catalyst (slurried in 1.2 g acetone) + TBA heated to 80 C; TBHP added from reservoir; 8.7 wt % total acetone. | | | | | | | |
| * 200 psig = 1,38 MPa | | | | | | | |

### Examples 16 through 21

Table III shows the effect of water removal on the decomposition of TBHP catalyzed by metal perhaloporphyrins. In Examples 16, 18 and 20, the t-butyl alcohol ("TBA"), the TBHP and the acetone used were wet, as described in footnote b to Table III. In Examples 17, 19 and 21, the TBA, TBHP and acetone were dry, as also described in footnote b to Table III. In Examples 17, 19 and 21, the cc's of oxygen evolved in the first hour were higher than in the corresponding Examples 16, 18 and 20, indicating that drying the components caused an increase in the effectiveness of the reaction system for decomposition of TBHP. The initial rate constant data confirm the superiority of Examples 17, 19 and 21 using dried components to Examples 16, 18 and 20 using undried components.

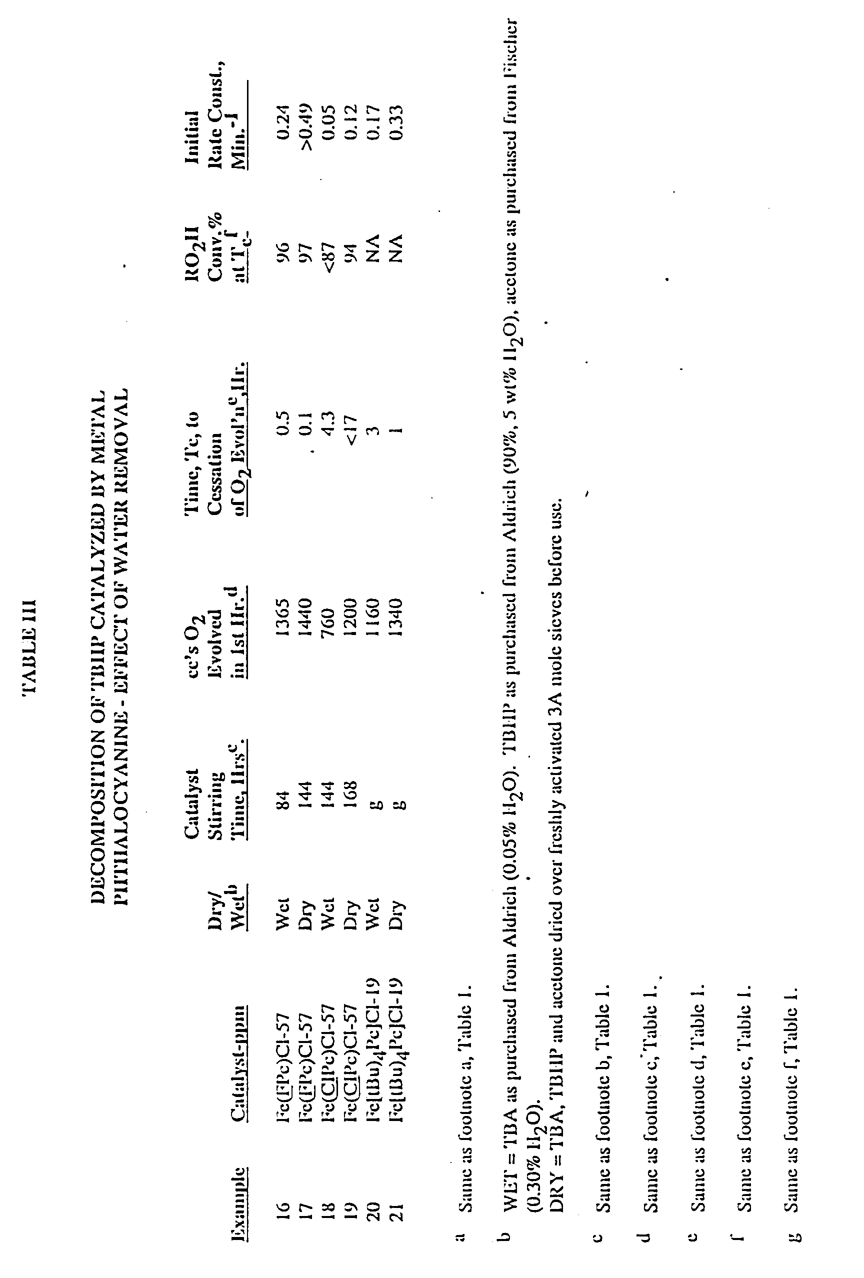

### Examples 22 through 25

Table IV shows the effect of drying TBHP on the subsequent decomposition of the TBHP. In Examples 22 through 24, the TBHP added to the reactor was wet, and in Example 25, the TBHP added to the reactor had been dried as described in footnote d to Table IV. In Example 23, wet TBHP was added to a reactor which contained in the reaction mixture a solid drying agent, activated molecular sieve (3A). Superior conversion of TBHP in a shorter reaction time was obtained in Example 23, where the reaction mixture contained drying agent, as compared with Example 22, where wet TBHP was added to a reaction mixture containing no drying agent. And superior conversion of TBHP was obtained in Example 25 where the TBHP was dried prior to addition to the reactor, as compared with Example 24 where the TBHP was added wet to the reaction mixture.

**TABLE IV**

| **TBHP DECOMPOSITION Effect of drying TBHP Fe[FPc]Cl catalyst @ 80 C** | | | | | | | |
|---|---|---|---|---|---|---|---|
| **EXAMPLE #** | **RUN #** | **CAT CONC ppm** | **% CONV of TBHP** | **TIME min** | **PRESSURE psig** | **TBHP Charge mole %** | **Acetone Mole %** **Actual** |
| 22 | 3917a | 59 | 85.3 | 180 | 0 | 30.80 | 11.6 |
| 23 | 3921b | 59 | 90.7 | 90 | 0 | 31.60 | 11.6 |
| 24 | 3953c | 89 | 95.6 | 60 | 0 | 29.18 | 17.7 |
| 25 | 3955d | 89 | 99.1 | 60 | 0 | 29.18 | 17.7 |

| | | | | | | | |
|---|---|---|---|---|---|---|---|
| a 5.4 g WET TBHP added to the reactor; the reactor contained catalyst (slurried in acetone) and TBA @ 80 C. | | | | | | | |
| b 5.4 g WET TBHP added to the reactor; the reactor contained catalyst (slurried in acetone) and TBA with 2 g activated molecular sieves (3A) @ 80 C. | | | | | | | |
| c 5.4 g WET TBHP, 1.0 g acetone and 7.2 g TBA heated to 80 C in a reactor; catalyst added as acetone slurry in 3 aliquots. | | | | | | | |
| d 5.4 g TBHP (dried using 3A mol sieves), 1.0 g acetone and 7.2 g TBA heated to 80 C in a reactor; catalyst added as acetone slurry in 3 aliquots. | | | | | | | |

### Examples 26 through 29

Table V shows the effect of water removal on the decomposition of TBHP catalyzed by metalloporphyrins. The catalysts used in these examples were metal complexes of porphyrin derivatives: in Examples 26 and 27, µ-oxo dimer of Fe^{III} meso-tetranitro-beta-octaethylporphyrin, and in Examples 28 and 29, Fe^{III} meso-(pentafluorophenyl-beta-octachloroporphyrin) chloride. More cc's of oxygen were evolved in the first hour in Example 26, where the reaction mixture was dry, compared to Example 27, where the reaction mixture was wet. And more cc's of oxygen were evolved in the first hour in Example 28, where the reaction mixture was dry, compared to Example 29 where the reaction mixture was wet.

**TABLE V**

| **DECOMPOSITION OF TBHP CATALYZED BY METALLOPORPHYRINS - EFFECT OF WATER REMOVAL** | | | | | | |
|---|---|---|---|---|---|---|
| **Example** | **Catalyst** | **Dry/Wet**^{**b**} | **cc's O**_{**2**} **Evolved in 1st Hr**^{**c**} | **Time, T**_{**c**}**, to Cessation of O**_{**2**} **Evol'n**^{**d**}**, hr.** | **RO2H Conv.% at T**_{**c**}^{**c**} | **Initial Rate Const.**, **Min.**^{**-1**} |
| 26 | [Fe(TNOEP)]₂O | Dry | 1380 | 1.5 | 95 | 0.27 |
| 27 | [Fe(TNOEP)]₂O | Wet | 1280 | 2 | 93 | 0.17 |
| 28 | Fe(TPPF₂₀Cl₈)Cl | Dry | 1350(est.) | >1 | NA | 0.28 |
| 29 | Fe(TPPF₂₀Cl₈)Cl | Wet | 1140 | <3 | NA | 0.03 |

| | | | | | | |
|---|---|---|---|---|---|---|
| ^{a} The catalyst, 0.6mg, was added to a solution of 13.8 grams TBHP in 18.1 grams TBA stirred at 80°C. The solution was stirred at 80°C for the designated time. Oxygen evolution was followed manometrically and the liquid product profile monitored by standardized glpc. | | | | | | |
| ^{b} Same as footnote b, Table 3. | | | | | | |
| ^{c} Same as footnote c, Table 1. | | | | | | |
| ^{d} Same as footnote d, Table 1. | | | | | | |
| ^{e} Same as footnote e, Table 1. | | | | | | |

### Examples 30 through 34

Table VI shows the effect of reaction pressure on TBHP decomposition using iron phthalocyanine chloride catalyst at 80°C. The pressure was varied from 0 to 1.38 MPa (0 to 200 psig) in five runs. Examples 30 and 31 show the effect of changing the pressure from 0 to 200 psig under wet conditions. The percent conversion of TBHP was higher at 0 psig than at 200 psig. Examples 32 and 33 show the effect of changing the pressure from 0 to 345 kPa (0 to 50 psig) under dry conditions. The percent conversion of TBHP was higher at 0 kPa (0 psig) than at 345 kPa (50 psig).

**TABLE VI**

| **TBHP DECOMPOSITION Effect of reaction pressure Fe[****F****Pc]Cl catalyst @ 80 C** | | | | | | | |
|---|---|---|---|---|---|---|---|
| **EXAMPLE #** | **RUN #** | **CAT CONC ppm** | **% CONV of TBHP** | **TIME min** | **PRESSURE psig** | **TBHP Charge mole %** | **TBHP State** |
| 30 | 3917a | 59 | 85.3 | 180 | 0 | 30.80 | WET |
| 31 | 3925a | 59 | 79.6 | 180 | 200* | 31.68 | WET |
| 32 | 4023b | 65 | 99.0 | 60 | 0 | 27.93 | DRY |
| 33 | 4021c | 83 | 97.4 | 60 | 50* | 26.78 | DRY |
| 34 | 4025d | 101 | 98.6 | 60 | 50* | 25.73 | DRY |

| | | | | | | | |
|---|---|---|---|---|---|---|---|
| a Catalyst (slurried in acetone) + TBA heated to 80 C; TBHP added from reservoir; N2 sweep @ 10 cc/min | | | | | | | |
| b TBA + TBHP + acetone heated to 80 C in reactor. Catalyst (slurried in acetone) added in 3 aliquots. | | | | | | | |
| c TBA + TBHP + acetone heated to 80 C in reactor. Catalyst (slurried in acetone) added in 4 aliquots; N2 swwep @ 10 cc/min | | | | | | | |
| d TBA + TBHP + acetone heated to 80 C in reactor. Catalyst (slurried in acetone) added in 3 aliquots; N2 swwep @ 10 cc/min | | | | | | | |
| * 200 psig = 1.38 MPa 50 psig = 345 kPa | | | | | | | |

### Example 35

### Synthesis of Fe(FPc)-O-Fe(FPc)

A quantity of 300mg of iron perfluorophthalocyanine, Fe(FPc), is stirred in 100ml of tetrahydrofuran for 12 days in the presence of air. After this time the mixture is filtered through a medium coarse glass fritted funnel and the filtrate evaporated to dryness then heated in vacuo overnight at 110°C. The UV/vis spectrum shows a λₘₐₓ at 628nm. The mass spectrum shows a signal at M=1728, corresponding to this µ-oxo dimer.

### Example 36

### Synthesis of Fe[(t-butyl)₄Pc]Cl

To a stirring solution of 6.5g of t-butylphthalic anhydride in 25ml of 1,2,4-trichlorobenzene is added 7.0g of urea, 0.2g of ammonium molybdate and finally 2.55g of anhydrous FeCl₃. The stirred mixture is heated to 190°C. and held there for 4hr after which time the solution is cooled and filtered. To the filtrate is added 25ml of petroleum ether (60-90°). After overnight this material is filtered to give dark blue microcrystalline material. The yield is 35% based on the starting t-butylphthalic anhydride, of Fe[(t-butyl)₄Pc]Cl. UV/vis(CHCl₃) 608,644 sh. 676 mm.

1.0g of the Fe[t-butyl)₄Pc]Cl is stirred in 20ml of acetone with 1.0g of NaN₃ for 12hr. The material is filtered and the filtrate evaporated to dryness. The solids are redissolved in chloroform and washed with H₂O then dried over sodium sulfate and evaporated to dryness. The yield is quantitative of Fe[(t-butyl)₄Pc]N₃ with a ν N-N in the IR spectrum of 2050 cm⁻¹.

### Examples 37 through 41

Table VII shows the effect of drying on the decomposition of t-butyl hydroperoxide (TBHP), solubilized in t-butyl alcohol (TBA), catalyzed by Co(acac)₂ at 80°C and 0 psig. TBHP was decomposed in Examples 37 to 40 using Co(acac)₂, and the effect of drying was determined by varying the water level in the runs from 4.3 wt.% ("wet" TBHP) to 1.2 and 1.1 wt.% ("1x dried" TBHP) to 0.1 wt.% ("2x dried" TBHP). The procedure used was as described in footnotes 1, 2 and 3 to Table VII. The percent conversion of TBHP was measured after 60 minutes. In Example 41, the decomposition reaction was run with no catalyst.

The data under the heading "% Conv of TBHP" in Table VII indicate the relative activity of the runs. The conversion of TBHP increased from 46.4% for the wet system to 90.3% for the twice dried system with 0.1 wt.% water. Comparison of Examples 37 through 40 shows that drying of the reaction mixture significantly increased the conversion of TBHP. The results from Example 41 (39.0% conversion with no catalyst) highlight the poor activity of Co(acac)₂ in a wet system (see, Example 37).

**TABLE VII**

| TBHP Decomposition Catalyzed by Co(acac)₂ at 80°C Effect of Drying TBHP | | | | | |
|---|---|---|---|---|---|
| Example No. | Catalyst Conc (ppm) | Water in Charge(wt%) | Time (min) | Pressure (psig) | % Conv. of TBHP |
| 37 ¹ | 89 | 4.3 (wet) | 60 | 0 | 46.4 |
| 38 | 89 | 1.2 (1x dried) | 60 | 0 | 88.2 |
| 39 | 89 | 0.1 (2x dried) | 60 | 0 | 90.3 |
| 40 ² | 83 | 1.1 (1x dried) | 60 | 0 | 77.3 |
| 41 ³ | 0 | 1.1 (1x dried) | 60 | 0 | 39.0 |

| | | | | | |
|---|---|---|---|---|---|
| ¹ For Examples 37, 38 and 39: 5.4 g TBHP + 7.2 g dried TBA in stainless steel reactor; purge with N₂; heat and stabilize at 80°C; add 4 aliquots of catalyst solution (674 ppm in dried TBA) at 0, 7, 14 and 21 minutes into the run. | | | | | |
| ² Same as "1", but the reactor charge contains 1 g dry acetone. | | | | | |
| ³ Same as "1", but the reactor charge contains 1 g dry acetone, and no catalyst. | | | | | |

### Examples 42 through 48

Table VIII shows the effect of drying on the decomposition of t-butyl hydroperoxide (TBHP), solubilized in t-butyl alcohol (TBA) and acetone, catalyzed by iron phthalocyanine (FePc) at 80°C and 0 psig. TBHP was decomposed in Examples 42 to 46 using FePc, and the effect of drying was determined by varying the water level in the runs from 3.8 wt.% ("wet" TBHP) to 1.8 wt.% ("1x dried" TBHP) to 0.05 wt.% ("2x dried" TBHP). In Example 47, water was added to a twice dried reaction mixture to yield 0.33 wt.% water. In Example 48, the reaction was run with "wet" TBHP with 2 g of activated molecular sieve drying agent in the reaction system.

**TABLE VIII**

| TBHP Decomposition Catalyzed by FePc at 80°C Effect of Drying TBHP | | | | | |
|---|---|---|---|---|---|
| Example No. | Catalyst Conc. (ppm) | Water in Charge(wt.%) | Pressure (psig) | % Conv. of TBHP | Remarks |
| 42 | 9 | 3.8 | 0 | 23.0 | wet TBHP |
| 43 | 9 | 3.8 | 0 | 19.9 | wet TBHP |
| 44 | 9 | 1.8 | 0 | 56.8 | 1x dried TBHP |
| 45 | 9 | 1.8 | 0 | 53.5 | 1x dried TBHP |
| 46 | 9 | 0.05 | 0 | 63.8 | 2x dried TBHP |
| 47 | 9 | 0.33 | 0 | 61.9 | 2x dried TBHP + 0.04g water |
| 48 | 9 | 3.8 | 0 | 40.5 | wet + 2g 3A mol.sieve added |
| 5.4g TBHP + 7.2g dried TBA + 1g dried acetone in stainless steel reactor; purge with N₂; heat and stabilize at 80°C; inject 0.6 ml catalyst solution (674 ppm in dried acetone) at time 0. Run for 60 min; cool down and analyze product. | | | | | |

The procedure used was as described in the footnote to Table VIII. The percent conversion of TBHP was measured after 60 minutes. The results in Table VIII further illustrate the positive effect of drying the reaction mixture on the percent conversion of TBHP. Conversion after 1 hour with the catalyst is approximately three times greater in the twice-dried system as compared to the wet system.

The results for the decomposition of TBHP catalyzed by Co(acac)₂ and the effect of drying on that reaction are further illustrated in Figures 1 through 6. Figures 1 and 2 represent hydroperoxide decomposition by the amount of O₂ (in cc's) evolved in the reaction plotted against the reaction time (in minutes). They dramatically show that as water is removed from the reaction mixture, Co(acac)₂ becomes an increasingly superior decomposition catalyst. It has been found that Co(acac)₂ has little or no catalytic activity under the reaction conditions described herein when the TBHP feed is contaminated with approximately 5 wt.% water (results not shown). In contrast, Figures 1 and 2 show that drying to approximately 1 wt.% or lower of water results in a very active catalyst. The data in Table IX further evidence this high catalytic activity of Co(acac)₂ for decomposition in dried conditions.

Figure 3 shows the dramatic increase in the decomposition rate for the Co(acac)₂-catalyzed decomposition of TBHP in TBA as water is removed from the reaction mixture. The Figure plots the O₂ evolved in the first minute of the reaction against the water concentration in the reaction mixture. The data show that the rate begins to rise rapidly as the water concentration is decreased below 1 wt.%. In fact, the initial reaction rate at 0.25 wt.% water in feed is five times greater than at 1 wt.% water in feed.

Figures 4 and 5 illustrate the effect of drying on TBHP decomposition catalyzed by Ru(acac)₃ by showing the results in wet and dried systems, respectively. TBHP was solubilized in TBA ; the reaction was run at 80°C; and activity was measured as a function of O₂ evolved (in mmoles) and plotted against reaction time (in hours). In Figure 5, it can be seen that, after a brief induction period, the decomposition reaction was observed to proceed extremely rapidly. The reaction was 98% complete after one hour.

Figure 6 illustrates the further positive effect that occurs when the combination of Co(acac)₂ and Ru(acac)₃ is used as the catalyst for TBHP decomposition in a dried system.

**TABLE IX**

| Effect of Added Water on Co(acac)₂ Catalyzed Conversion of TBHP to TBA | | | | | | | | |
|---|---|---|---|---|---|---|---|---|
| | O₂ Evolved, cc, after time period, min | | | | | | TBHP | TBA |
| Water wt.% | 5 | 7 | 20 | 30 | 60 | 1440 | Conv. (%) | (Mol %) |
| 0.25 | 1310 | 1330 | 1380 | 1390 | 1410 | 1440 | 95 | 96 |
| 1.06 | 410 | 480 | 720 | 820 | 950 | 1320 | 85 | 92 |
| 1.54 | 420 | 480 | 690 | 770 | 870 | 1280 | 78 | 92 |
| 2.01 | 300 | 370 | 610 | 720 | 840 | 1085 | 74 | 88 |
| 2.48 | 270 | 340 | 500 | 540 | 610 | 950 | 59 | 80 |
| 2.95 | 160 | 200 | 260 | 280 | 320 | 670 | 30 | 71 |
| 4.11 | - NR - | - NR - | - NR - | - NR - | - NR - | - NR - | --- | --- |
| The catalyst, 0.6 mg, was added to a stirred solution of TBHP (13.9g), TBA (18.1g), and water (added to the concentration designated in the Table) at 80°C. Oxygen evolution was monitored manometrically over time. The liquid phase was analyzed periodically by standardized GLPC and a final analysis done at 24 hours - results reported in the Table. | | | | | | | | |

### Example 49

A solution from a concentrated cyclohexane oxidate comprising cyclohexyl hydroperoxide (10%), cyclohexanone (58%) and cyclohexanol (31%) and small amounts of other organic materials including cyclohexane is dried for 72 hours over freshly activated molecular sieve. The dried reaction mixture is then catalytically decomposed using Co(acac)₂. The rate of decomposition of the dried reaction mixture is more than twice that of an identical undried sample.

### Example 50

A concentrated cyclohexane solution comprising 10.5% cyclohexyl hydroperoxide, 2.5% cyclohexanol, 1% cyclohexanone and 3.5% other oxidation products is generated by non-catalytic oxidation of cyclohexane in a passivated reactor. The reaction mixture is dried over 3A molecular sieves. The dried reaction mixture is then catalytically decomposed using Co(acac)₂. Decomposition of the hydroperoxide in this mixture occurs far more rapidly when the mixture is thoroughly dried over 3A molecular sieves than when the undried material is used.

## Claims

1. Method for decomposing organic hydroperoxides which comprises drying a reaction mixture comprising an organic hydroperoxide, an organic solvent for said hydroperoxide, and water, or drying the organic hydroperoxide or the organic solvent, thereby to obtain a dried reaction mixture in which said water comprises 1 weight percent or less of said dried reaction mixture, and contacting said dried reaction mixture with a metal organic ligand catalyst for said decomposing.

2. Method according to Claim 1 which comprises drying said reaction mixture such that said water comprises 0.5 weight percent or less of said dried reaction mixture, preferably 0.25 weight percent or less, and in particular 0.1 weight percent or less of said dried reaction mixture.

3. Method according to Claim 1 or 2, wherein said metal organic ligand catalyst comprises ligand selected from phthalocyanines, other tetraazamacrocycles, porphyrins, porphenes, porphycenes, 1,3-bis(arylimino)isoindolines, acetylacetonates, Schiff bases, halogenated mono-, bi-, tri- and tetradentate ligand systems, or combinations thereof, and wherein said metal is selected from chromium, manganese, iron, ruthenium and cobalt.

4. Method according to Claim 3 wherein said catalyst comprises metal acetylacetonate or mixtures thereof, preferably cobalt acetylacetonate or ruthenium acetylacetonate, or a mixture of ruthenium acetylacetonate and cobalt acetylacetonate.

5. Method according to Claim 3 wherein said catalyst comprises a metallophthalocyanine or a metalloporphyrin.

6. Method according to Claim 5 wherein hydrogen atoms of said phthalocyanine or porphyrin have been replaced with electron-withdrawing atoms or groups, preferably selected from halogen, cyano, nitro and halocarbyl.

7. Method according to Claim 6 wherein said catalyst comprises a metallohalophthalocyanine or a metallohaloporphyrin, preferably a metalloperhalophthalocyanine or a metalloperhaloporphyrin.

8. Method according to Claim 3 wherein said catalyst comprises a mixture of metallophthalocyanine and metal acetylacetonate, preferably a mixture of iron phthalocyanine and cobalt acetylacetonate.

9. Method according to any of Claims 1 to 8, wherein said hydroperoxide comprises an alkylhydroperoxide, preferably t-butyl hydroperoxide or cyclohexyl hydroperoxide.

10. Method according to any of Claims 1 to 9, wherein said solvent is a hydroxyl-containing organic compound, preferably t-butanol or cyclohexanol.

11. Method according to any of Claims 1 to 10, wherein said reaction mixture additionally comprises an organic co-solvent for said hydroperoxide, and preferably said co-solvent comprises a ketone.

12. Method according to any of Claims 1 to 11, wherein said contacting occurs at a temperature in the range from 25°C to 150°C and at a total pressure not greater than 3.45 MPa (500 psig).

13. Method according to any of Claims 1 to 12, wherein said drying of said reaction mixture is in the absence of said catalyst.

14. Method according to any of Claims 1 to 12, wherein said drying of said reaction mixture is in the presence of said catalyst.

15. Method according to Claim 14 wherein said hydroperoxide, said solvent, said water and said catalyst are contacted with a granular solid drying agent, thereby to remove water from said reaction mixture and decompose said hydroperoxide.

## Patentansprüche

1. Verfahren zur Zersetzung organischer Hydroperoxide, das das Trocknen eines Reaktionsgemisches, das ein organisches Hydroperoxid, ein organisches Lösungsmittel für das Hydroperoxid und Wasser enthält, oder das Trocknen des organischen Hydroperoxids oder des organischen Lösungsmittels, wobei man ein getrocknetes Reaktionsgemisch erhält, in dem Wasser 1 Gew.-% oder weniger des getrockneten Reaktionsgemisches ausmacht, und das Kontaktieren des getrockneten Reaktionsgemisches mit einem Metall-organischer-Ligand-Katalysator für die Zersetzung umfaßt.

2. Verfahren nach Anspruch 1, das das Trocknen des Reaktionsgemisches umfaßt, so daß das Wasser 0,5 Gew.-% oder weniger des getrockneten Reaktionsgemisches, vorzugsweise 0,25 Gew.-% oder weniger und insbesondere 0,1 Gew.-% oder weniger des getrockneten Reaktionsgemisches ausmacht.

3. Verfahren nach Anspruch 1 oder 2, wobei der Metallorganischer-Ligand-Katalysator einen Liganden umfaßt, der unter Phthalocyaninen, anderen Tetraazamacrocyclen, Porphyrinen, Porphenen, Porphycenen, 1,3-Bis(arylimino)isoindolinen, Acetylacetonaten, Schiffschen Basen, halogenierten ein-, zwei-, drei- und vierzähnigen Ligandensystemen oder Kombinationen daraus ausgewählt ist, und wobei das Metall unter Chrom, Mangan, Eisen, Ruthenium und Cobalt ausgewählt ist.

4. Verfahren nach Anspruch 3, wobei der Katalysator Metallacetylacetonat oder Gemische davon, vorzugsweise Cobaltacetylacetonat oder Rutheniumacetylacetonat oder ein Gemisch aus Rutheniumacetylacetonat und Cobaltacetylacetonat, enthält.

5. Verfahren nach Anspruch 3, wobei der Katalysator ein Metallophthalocyanin oder ein Metalloporphyrin enthält.

6. Verfahren nach Anspruch 5, wobei Wasserstoffatome des Phthalocyanins oder Porphyrins durch Elektronen abziehende Atome oder Gruppen, die vorzugsweise unter Halogen, Cyan, Nitro und Halogencarbyl ausgewählt sind, ersetzt sind.

7. Verfahren nach Anspruch 6, wobei der Katalysator ein Metallohalogenphthalocyanin oder ein Metallohalogenporphyrin und vorzugsweise ein Metalloperhalogenphthalocyanin oder ein Metalloperhalogenporphyrin enthält.

8. Verfahren nach Anspruch 3, wobei der Katalysator ein Gemisch aus einem Metallophthalocyanin und einem Metallacetylacetonat und vorzugsweise ein Gemisch aus Eisenphthalocyanin und Cobaltacetylacetonat enthält.

9. Verfahren nach einem der Ansprüche 1 bis 8, wobei das Hydroperoxid ein Alkylhydroperoxid und vorzugsweise tert.-Butylhydroperoxid oder Cyclohexylhydroperoxid umfaßt.

10. Verfahren nach einem der Ansprüche 1 bis 9, wobei es sich bei dem Lösungsmittel um eine Hydroxyl enthaltende organische Verbindung und vorzugsweise um tert.-Butanol oder Cyclohexanol handelt.

11. Verfahren nach einem der Ansprüche 1 bis 10, wobei das Reaktionsgemisch zusätzlich ein organisches Co-Lösungsmittel für das Hydroperoxid umfaßt und das Co-Lösungsmittel vorzugsweise ein Keton umfaßt.

12. Verfahren nach einem der Ansprüche 1 bis 11, wobei das Kontaktieren bei einer Temperatur im Bereich von 25°C bis 150°C unter einem Gesamtdruck von nicht mehr als 3,45 MPa (500 psig) erfolgt.

13. Verfahren nach einem der Ansprüche 1 bis 12, wobei das Trocknen des Reaktionsgemisches in Abwesenheit des Katalysators erfolgt.

14. Verfahren nach einem der Ansprüche 1 bis 12, wobei das Trocknen des Reaktionsgemisches in Gegenwart des Katalysators erfolgt.

15. Verfahren nach Anspruch 14, wobei das Hydroperoxid, das Lösungsmittel, das Wasser und der Katalysator mit einem körnigen festen Trockenmittel in Kontakt gebracht werden, wobei das Wasser aus dem Reaktionsgemisch entfernt und das Hydroperoxid zersetzt wird.

## Revendications

1. Procédé de décomposition d'hydroperoxydes organiques, caractérisé en ce que l'on sèche un mélange réactionnel comprenant un hydroperoxyde organique, un solvant organique pour ledit hydroperoxyde et de l'eau, ou le séchage de l'hydroperoxyde organique ou du solvant organique, de façon à obtenir un mélange de réaction séché dans lequel l'eau précitée constitue de 1% en poids ou moins encore du mélange de réaction séché précité et la mise en contact dudit mélange de réaction séché avec un catalyseur à ligand organique de métal pour ladite décomposition.

2. Procédé suivant la revendication 1, caractérisé en ce que l'on sèche ledit mélange réactionnel en une manière telle que ladite eau constitue de 0,5% en poids ou moins dudit mélange de réaction séché, de préférence, de 0,25% ou moins et, bien mieux encore, de 0,1% ou moins dudit mélange de réaction séché.

3. Procédé suivant la revendication 1 ou 2, caractérisé en ce que ledit catalyseur à ligand organique de métal comprend un ligand choisi parmi les phtalocyanines, d'autres tétraazamacrocycles, des porphyrines, des porphènes, des porphycènes, des 1,3-bis(arylimino)isoindolines des acétylacétonates, des bases de Schiff, des systèmes à ligands halogénés monodentates, bidentates, tridentates et tétradentates, ou leurs combinaisons et en ce que ledit métal est choisi parmi le chrome, le manganèse, le fer, le ruthénium et le cobalt.

4. Procédé suivant la revendication 3, caractérisé en ce que ledit catalyseur comprend un acétylacétonate de métal ou des mélanges de tels composés, de préférence, de l'acétylacétonate de cobalt ou de l'acétylacétonate de ruthénium, ou un mélange d'acétylacétonate de ruthénium et d'acétylacétonate de cobalt.

5. Procédé suivant la revendication 3, caractérisé en ce que ledit catalyseur comprend une métallophtalocyanine ou une métalloporphyrine.

6. Procédé suivant la revendication 5, caractérisé en ce que des atomes d'hydrogène de ladite phtalocyanine ou de ladite porphyrine ont été remplacés par des atomes ou groupes soutirant des électrons, de préférence, des atomes d'halogènes, des radicaux cyano, nitro et halocarbyle.

7. Procédé suivant la revendication 6, caractérisé en ce que ledit catalyseur comprend une métallohalophtalocyanine ou une métallohaloporphyrine, de préférence, une métalloperhalophtalocyanine ou une métalloperhaloporphyrine.

8. Procédé suivant la revendication 3, caractérisé en ce que le catalyseur comprend une métallophtalocyanine et un acétylacétonate de métal, de préférence, un mélange de phtalocyanine de fer et d'acétylacétonate de cobalt.

9. Procédé suivant l'une quelconque des revendications 1 à 8, caractérisé en ce que ledit hydroperoxyde est constitué par un hydroperoxyde d'alkyle, de préférence, l'hydroperoxyde de t-butyle, ou l'hydroperoxyde de cyclohexyle.

10. Procédé suivant l'une quelconque des revendications 1 à 9, caractérisé en ce que ledit solvant est un composé organique contenant des radicaux hydroxyle, de préférence, le t-butanol ou le cyclohexanol.

11. Procédé suivant l'une quelconque des revendications 1 à 10, caractérisé en ce que ledit mélange de réaction comprend en outre un cosolvant organique pour ledit hydroperoxyde et, de préférence, ledit cosolvant comprend une cétone.

12. Procédé suivant l'une quelconque des revendications 1 à 11, caractérisé en ce que ladite mise en contact s'opère à une température qui varie de 25°C à 150°C et sous une pression totale non supérieure à 3,45 MPa (500 psig).

13. Procédé suivant l'une quelconque des revendications 1 à 12, caractérisé en ce que ledit séchage dudit mélange réactionnel s'effectue en l'absence dudit catalyseur.

14. Procédé suivant l'une quelconque des revendications 1 à 12, caractérisé en ce que ledit séchage dudit mélange réactionnel s'effectue en présence dudit catalyseur.

15. Procédé suivant la revendication 14, caractérisé en ce que ledit hydroperoxyde, ledit solvant, ladite eau et ledit catalyseur sont mis en contact avec un agent de séchage solide granulaire, de manière à éliminer l'eau dudit mélange réactionnel et à décomposer ledit hydroperoxyde.
